# EUROPEAN PATENT APPLICATION

(11) **EP 2 615 458 A1**
(43) Date of publication of application: **17.07.2013**
(21) Application number: 11823245.3
(22) Date of filing: 06.09.2011
(51) Int. Cl.: G01N 33/68, G01N 33/53

(54) **METHOD FOR DIAGNOSING ALZHEIMER'S DISEASE USING BLOOD-SOLUBLE GPVI**

(30) Priority: 07.09.2010 JP 2010200398
(71) Applicant: Mochida Pharmaceutical Co., Ltd., Tokyo 160-8515 (JP)
(72) Inventor: NAITOH, Katsuki, Tokyo 160-8515 (JP); HOSAKA, Yoshitaka, Tokyo 160-8515 (JP)
(74) Representative: von Kreisler Selting Werner
(86) International application number: PCT/JP2011/004990
(87) International publication number: WO 2012/032766

(57) **Abstract**

Provided are an agent for noninvasively and easily diagnosing Alzheimer's disease, a method for diagnosing Alzheimer's disease through the measurement, and a measurement kit therefor. The present invention relates to a reagent for measuring soluble GPVI (sGPVI) in a human body fluid; an agent for specifically diagnosing Alzheimer's disease, said agent comprising a reagent for measuring the activation of platelets or coagulation-fibrinolysis system in said human body fluid; and a method for specifically diagnosing Alzheimer's disease, said method comprising a step for measuring the concentration of sGPVI in a collected human body fluid and a step for measuring the activation of platelets or coagulation-fibrinolysis system in said human body fluid.

## Description

### TECHNICAL FIELD

The present invention relates to a reagent for measuring the concentration of soluble GPVI in blood; an agent for noninvasively and easily diagnosing Alzheimer's disease, the agent comprising a reagent for measuring the activation of platelets or coagulation-fibrinolysis system with a platelet activation marker or a coagulation-fibrinolysis system activation marker; a method for diagnosing Alzheimer's disease through such measurement; and a measurement kit therefor.

### BACKGROUND ART

Increase in patients with dementia has become a major social problem accompanied with the aging society. According to the estimation of dementia patients in 2005, the dementia patients throughout the world are considered to be 24.3 million people and it is said that 4.6 million new patients occur each year. Approximately 70% of those are regarded as Alzheimer's dementia. As a symptom of Alzheimer's dementia, "core symptoms" including loss in memory or conversation and in a daily living activity, as well as "peripheral symptoms" such as wandering, delusions, and violent behavior can be found. The Alzheimer's dementia when compared to other disease is a serious disease that would significantly reduce the QOL of not only patient oneself, of course, but also the patient's family. Therefore, there is an urgent need to make a diagnosis of Alzheimer's disease as quickly and exactly as possible, and to develop an effective prevention method and a therapy method.

In the United States of America in 2004, with an aim to establish an effective therapeutic method of Alzheimer's disease, an international clinical study ADNI (Alzheimer Disease Neuroimaging Initiative) started to objectively measure progression of Alzheimer's disease using biological samples, such as patient's blood and cerebrospinal fluid, and image diagnosis. Also in Japan in 2007, J-ANDI was established and it has been planned to perform a follow-up survey for 2-3 years about brain imaging and blood or cerebrospinal fluid.

A method for the diagnosis of Alzheimer's disease includes neuropsychological tests such as DSM-IV or NINCD-ARDA and ICD-10. These test methods are simple and do not require special instruments, but they have disadvantages that a long observation period is required and there is a poor reproducibility depending on the mood of patients. In addition, since the problems such as differences between facilities are pointed out and standardization to diagnose Alzheimer's disease has not been achieved yet, biochemical biomarkers that can be objectively evaluated are desired.

With advances in image diagnosis in recent years, a number of diagnostic methods for Alzheimer's disease using image diagnosis have also been reported. For example, CT/MRI is a diagnostic method to detect morphological abnormalities of the brain, and SPECT is a diagnostic method to observe a functional abnormality of the brain by measuring cerebral blood flow. Many diagnostic methods using PET have also been developed. In addition, by measuring the abnormal glucose metabolism in the brain, FDG-PET is now available for the diagnosis of Alzheimer's disease, and by imaging the accumulation of amyloid beta in the brain in the living body, amyloid PET is now available for the diagnosis of Alzheimer's disease. These advances in image diagnosis method are said to be a breakthrough in clinical studies of Alzheimer's disease in recent years.
However, these image diagnosis methods are not simple because they require special machines and facilities. Moreover, PET and SPECT are a highly invasive diagnostic method because a radioactive isotope is administered into the living body though it is in a very small amount.

A promising biochemical diagnostic biomarker of Alzheimer's disease is to measure phosphorylated tau and amyloid β in the brain (see Non-Patent Document 1). Particularly, phosphorylated tau in the brain reflects the nature of histopathological aspects of Alzheimer's disease, and it has been reported that such measurement of phosphorylated tau in the brain is a diagnostic method with high sensitivity and specificity. However, because such a method to measure these biomarkers requires the cerebrospinal fluid of patients, this method is a highly invasive diagnostic method as well.

For the profile of the diagnostic agent of Alzheimer's disease, reliability/reproducibility/noninvasiveness/simplicity/low cost have been desired, but there is no diagnostic agent that meets all these conditions at the moment. The revised draft of diagnostic criteria for Alzheimer's disease has been reported recently in the Alzheimer's Association International Conference on Alzheimer's disease 2010 (AAICAD2010). In the revised draft, there are newly pointed out the following points: 1) It began to deepen understanding of distinction of Alzheimer's dementia and non-Alzheimer's dementia, and overlap thereof, 2) Coexistence of Alzheimer's disease and cerebrovascular disease is common, 3) Many things are also known about non-Alzheimer's dementia such as dementia from Levi body disease and frontotemporal dementia such as Pick's disease, 4) In the three stages of Alzheimer's disease that are generally considered to currently exist (preclinical Alzheimer's disease, mild cognitive impairment (MCI) due to Alzheimer's disease, and Alzheimer's disease dementia), the role of biomarkers differs in each of the three stages, but there is the need for clinical evaluation tools such as biomarkers.

Glycoprotein VI (GPVI)) present on the platelet membrane is a collagen receptor of platelets, and it has been shown to have a central role in collagen-induced platelet activation. In addition, anti-mouse GPVI antibody has been reported to exhibit antithrombotic activity by specifically inhibiting collagen-induced platelet aggregation without causing a remarkable prolongation of bleeding time. Further, anti-human GPVI antibody has been reported to exhibit antithrombotic activity without causing a remarkable prolongation of bleeding time as well (see Patent Document 1). On the other hand, GPVI is also known to exist as soluble GPVI in body fluids such as blood (hereinafter sometimes described as sGPVI).

The present inventors have reported a specific and highly sensitive method for measuring sGPVI in human body fluids (see Patent Document 2). And concentration of plasma sGPVI is increased in patients with angina pectoris, acute myocardial infarction, heart disease, cerebral infarction, and dementia, and thus sGPVI was confirmed to be useful as a platelet activation marker. In addition, a measurement system for sGPVI in human body fluids and measurement results of healthy individuals were reported (see Patent Document 3).
In recent years, it has been also reported that the concentration in plasma sGPVI rises with age, but does not rise in patients with Alzheimer's disease (see Non-Patent Document 4). However, details about the relationship between concentration of sGPVI in human body fluids and Alzheimer's disease have not been fully examined and there has been no report that sGPVI will rise in Alzheimer's disease. A number of methods for diagnosing Alzheimer's disease have been reported so far, but they have not reached the level that can be satisfactory, due to (1) poor reproducibility/reliability, (2) need of specialized instruments, and (3) high invasiveness. Accordingly, development of biomarkers that can be used for easy diagnosis using blood or the like has been desired.
Further, as an animal model of Alzheimer's disease exhibiting a cognitive dysfunction, amyloid precursor protein transgenic mice (hereinafter described as APP-Tg mice) are produced (Non-Patent Document 5), but whether or not sGPVI increases in the blood of these mice is not known.

### PRIOR ART DOCUMENTS

### PATENT DOCUMENTS

Patent Document 1: WO 2005/111083
Patent Document 2: WO 2007/116779
Patent Document 3: JP 2008-249552

### NON-PATENT DOCUMENTS

Non-Patent Document 1: Geert De Meyer, et al., Arch. Neurol., 67, 949-956 (2010)
Non-Patent Document 2: Hiroyuki Arai, et al., Folia Pharmacol. Jpn 135, 3-7 (2010)
Non-Patent Document 3: Tsuj i M., et al., J. Biol. Chem., 272, 23528-23531 (1997)
Non-Patent Document 4: Christoph Laske, et al., J. Psychiatric Res., 42 (2008) 746-751
Non-Patent Document 5: Marcus A. Westerman, et al., J. Neurosci. 2002, 22, 1858-1867

### SUMMARY OF THE INVENTION

### PROBLEMS TO BE SOLVED BY THE INVENTION

The present invention provides an agent for noninvasively and easily diagnosing Alzheimer's disease, a method for diagnosing Alzheimer's disease through the measurement, and a measurement kit therefor.

### MEANS FOR SOLVING THE PROBLEMS

The present inventors performed an exhaustive screening of diseases and confirmed that sGPVI existed at a high concentration in the blood of patients with Alzheimer's disease by a screening method using age-matched clinical specimens. Furthermore, the present inventors found for the first time that sGPVI was produced in patients with Alzheimer's disease in large quantities without excessive activation of platelets or fibrinolysis-coagulation system unlike thrombotic diseases or thrombosis.
That is, when the concentration of sGPVI in the serum or plasma of patients with Alzheimer's disease and patients with thrombotic disease, and the platelet activation marker as well as the coagulation-fibrinolysis system activation marker were measured, the present inventors have found that the concentration of sGPVI in patients with thrombotic disease, and the platelet activation marker or the coagulation-fibrinolysis system activation marker were increased, but in Alzheimer's patients only the concentration of sGPVI was increased without accompanying rise in the platelet activation marker and in the coagulation-fibrinolysis system activation marker. And the inventors of the present application have found a non-invasive, simple, and specific method for diagnosing Alzheimer's disease using the body fluids such as serum and plasma, and completed the present invention.

That is, the present invention relates to a reagent for measuring sGPVI in a human body fluid, preferably a specific diagnostic or screening agent comprising the reagent and a reagent for measuring the activation of platelets or coagulation-fibrinolysis system in the human body fluid, or a kit for use in diagnosis or screening of Alzheimer's disease, i.e. a diagnostic or screening kit.
In addition, the present invention relates to a method for determining or diagnosing Alzheimer's disease specifically, the method comprising a process for measuring the concentration of sGPVI in a collected human body fluid, preferably a method for determining or diagnosing Alzheimer's disease specifically, the method comprising the process and a process for measuring the activation of platelets or coagulation-fibrinolysis system in the human body fluid, or a method for screening patients with Alzheimer's disease or patients with the possibility of developing Alzheimer's disease, i.e. patients at risk of Alzheimer's disease.

The present invention will be described in more detail as follows.
(1) A diagnostic agent for Alzheimer's disease or a diagnostic kit for Alzheimer's disease, comprising a reagent for measuring soluble GPVI (sGPVI) in a human body fluid.
(2) A diagnostic agent for Alzheimer's disease or a diagnostic kit for Alzheimer's disease, comprising a reagent for measuring sGPVI in a human body fluid and a reagent for measuring the activation of platelets or coagulation-fibrinolysis system in the human body fluid.
(3) The diagnostic agent or the diagnostic kit according to the above (1) or (2), wherein the reagent for measuring sGPVI comprises a substance that binds specifically to GPVI.
(4) The diagnostic agent or the diagnostic kit according to the above (3), wherein the substance that binds specifically to GPVI is an anti-GPVI antibody.
(5) The diagnostic agent or the diagnostic kit according to the above (4), wherein the anti-GPVI antibody is an antibody that binds specifically to domain 1 of GPVI, preferably loop 2 or loop 5, more preferably loop 2 of GPVI and/or domain 2 of GPVI, preferably loop 9 or loop 11, more preferably loop 9 of GPVI.

(6) The diagnostic agent or the diagnostic kit according to any one of the above (1) to (5), wherein the reagent for measuring sGPVI in a human body fluid comprises an antibody that specifically binds to loop 2 of GPVI as a non-immobilized antibody and an anti-GPVI antibody that specifically binds to loop 9 of GPVI as an immobilized antibody.
(7) The diagnostic agent or the diagnostic kit according to any one of the above (1) to (6), wherein the reagent for measuring sGPVI in a human body fluid is for the measurement by a sandwich immunoassay.
(8) The diagnostic agent or the diagnostic kit according to the above (7), wherein the sandwich immunoassay is a detection system using a biotinylated non-immobilized antibody and a poly-HRP-labeled streptavidin.
(9) The diagnostic agent or the diagnostic kit according to any one of the above (1) to (8), wherein the reagent for measuring the activation of platelets or coagulation-fibrinolysis system is a reagent for measuring a platelet activation marker and/or a coagulation-fibrinolysis system activation marker.
(10) The diagnostic agent or the diagnostic kit according to the above (9), wherein the platelet activation marker is soluble P-selectin (sP-selectin).
(11) The diagnostic agent or the diagnostic kit according to the above (9), wherein the coagulation-fibrinolysis system activation marker is a fibrinogen degradation product (FDP) or D-dimer in serum.
(12) The diagnostic agent or the diagnostic kit according to any one of the above (1) to (11), wherein the human body fluid is human blood, preferably serum or plasma.

(13) A method for determining or diagnosing Alzheimer's disease or screening patients with Alzheimer's disease or patients with the possibility of developing Alzheimer's disease, comprising a process for measuring the concentration of soluble GPVI (sGPVI) in a collected human body fluid.
(14) A method for determining or diagnosing Alzheimer's disease or screening patients with Alzheimer's disease or patients with the possibility of developing Alzheimer's disease, comprising a process for measuring the concentration of soluble GPVI (sGPVI) in a collected human body fluid and a process for measuring the activation of platelets or coagulation-fibrinolysis system in the human body fluid.
(15) The method according to the above (13) or (14), further comprising a process for confirming that the concentration of sGPVI in the human body fluid is a higher value than the control value.
(16) The method according to the above (13) or (14), further comprising a process for confirming that the concentration of sGPVI in the human body fluid is a higher value than the control value, and the value of the activation of platelets or coagulation-fibrinolysis system is not a higher value than the control value.
(17) The method according to any one of the above (13) to (15), wherein the process for measuring the concentration of sGPVI is a method using an anti-GPVI antibody.
(18) The method according to the above (17), wherein the anti-GPVI antibody is an antibody that specifically binds to domain 1 of GPVI, preferably loop 2 or loop 5, more preferably loop 2 of GPVI and/or an antibody that specifically binds to domain 2 of GPVI, preferably loop 9 or loop 11, more preferably loop 9 of GPVI.

(19) The method according to any one of the above (13) to (18), wherein the process for measuring the activation of platelets or coagulation-fibrinolysis system is a method for measuring a platelet activation marker and/or a coagulation-fibrinolysis system activation marker.
(20) The method according to the above (19), wherein the platelet activation marker is for the measurement of sP-selectin.
(21) The method according to the above (19), wherein the coagulation-fibrinolysis system activation marker is for the measurement of a fibrinogen degradation product (FDP) or D-dimer in serum.
(22) The method according to any one of the above (13) to (21), wherein the human body fluid is human blood, preferably serum or plasma.

(23) A kit for carrying out the methods according to the above (13) to (22), comprising an antibody that specifically binds to soluble GPVI.
(24) A method for predicting the onset of Alzheimer's disease by the methods according to the above (13) to (22).

### EFFECTS OF THE INVENTION

By using a diagnostic agent or a diagnostic kit of the present invention using a specimen of blood or the like, Alzheimer's disease can be diagnosed noninvasively and easily without the need for specialized instruments. In addition, a method using a diagnostic agent or a diagnostic kit of the present invention shows a high reproducibility and is able to specifically diagnose Alzheimer's disease. Moreover, determination and diagnosis in the present invention are highly objective based on the measurement values.
The method of the present invention can diagnose Alzheimer's disease easily by collecting a body fluid such as blood, etc., which makes it possible to diagnose a large number of subjects quickly in a short period of time, i.e., the screening becomes possible. In addition, since the measurement according to the method of the present invention is high in specificity, reproducibility, and objectivity, for example, performance of the method of the present invention on a regular basis makes it possible to early detect Alzheimer's disease, determine the stage or progression, evaluate the onset risk and predict the onset. Furthermore, combination with other detection methods as needed makes it possible to differentiate it from non-Alzheimer's disease dementia, for example, dementia from Levi body disease and frontotemporal dementia such as Pick's disease, in particular, vascular dementia, that is, it is also possible to distinguish the pathogenesis of dementia.

### BRIEF DESCRIPTION OF THE DRAWINGS

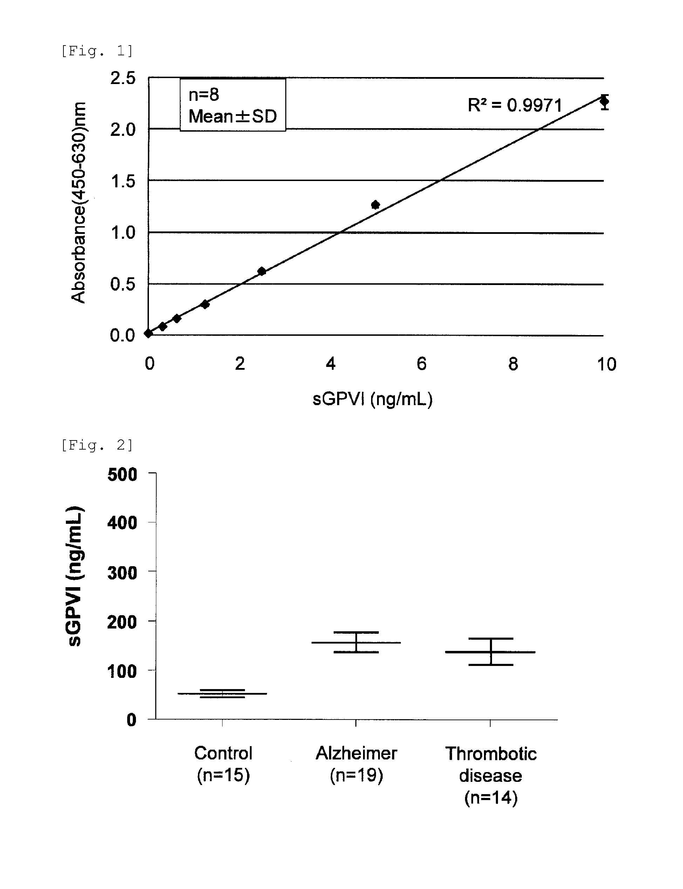

FIG. 1 is a graph of the standard curve of the absorbance and the concentrations (ng/mL) of sGPVI by the reagent for measuring the sGPVI in accordance with the present invention, using a recombinant human sGPVI-His.
FIG. 2 is a comparative graph of the concentrations (ng/mL) of plasma sGPVI between Alzheimer's patient samples (center of FIG. 2) and thrombotic patient samples (right side of FIG. 2) using a control sample (left side of FIG. 2) of healthy individuals. The long line in the figure shows the average value and the upper and lower bars indicate a standard error (SE).

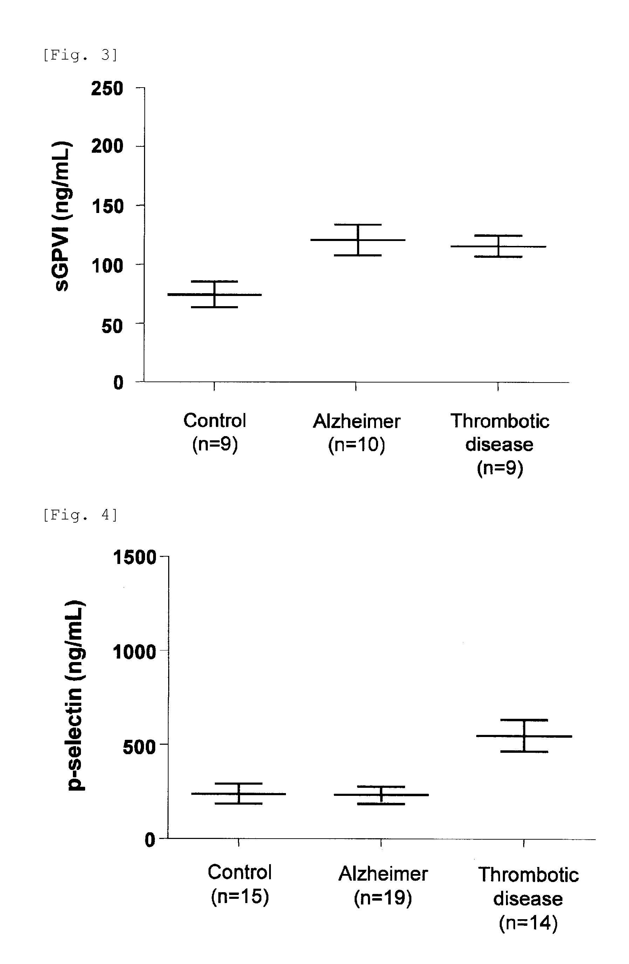

FIG. 3 is a comparative graph of the concentrations (ng/mL) of serum sGPVI between Alzheimer's patient samples (center of FIG. 3) and thrombotic patient samples (right side of FIG. 3) using a control sample (left side of FIG. 3) of healthy individuals. The long line in the figure shows the average value and the upper and lower bars indicate a standard error (SE).
FIG. 4 is a comparative graph of the concentrations (ng/mL) of plasma sP-selectin between Alzheimer's patient samples (center of FIG. 4) and thrombotic patient samples (right side of FIG. 4) using a control sample (left side of FIG. 4) of healthy individuals. The long line in the figure shows the average value and the upper and lower bars indicate a standard error (SE).

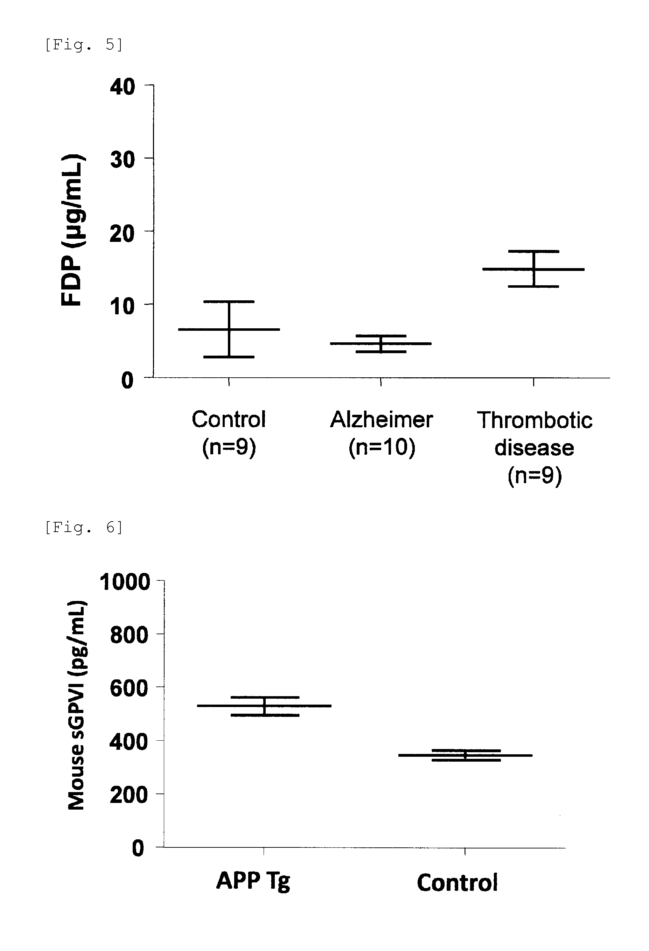

FIG. 5 is a comparative graph of the concentrations (µg/mL) of serum FDP between Alzheimer's patient samples (center of FIG. 5) and thrombotic patient samples (right side of FIG. 5) using a control sample (left side of FIG. 5) of healthy individuals. The long line in the figure shows the average value and the upper and lower bars indicate a standard error (SE).
FIG. 6 is a comparative graph of the concentrations (pg/mL) of plasma sGPVI in APP-Tg mice samples (left side of FIG. 6) using a control sample (right side of FIG. 6) of wild type mice. The long line in the figure shows the average value and the upper and lower bars indicate a standard error (SE).

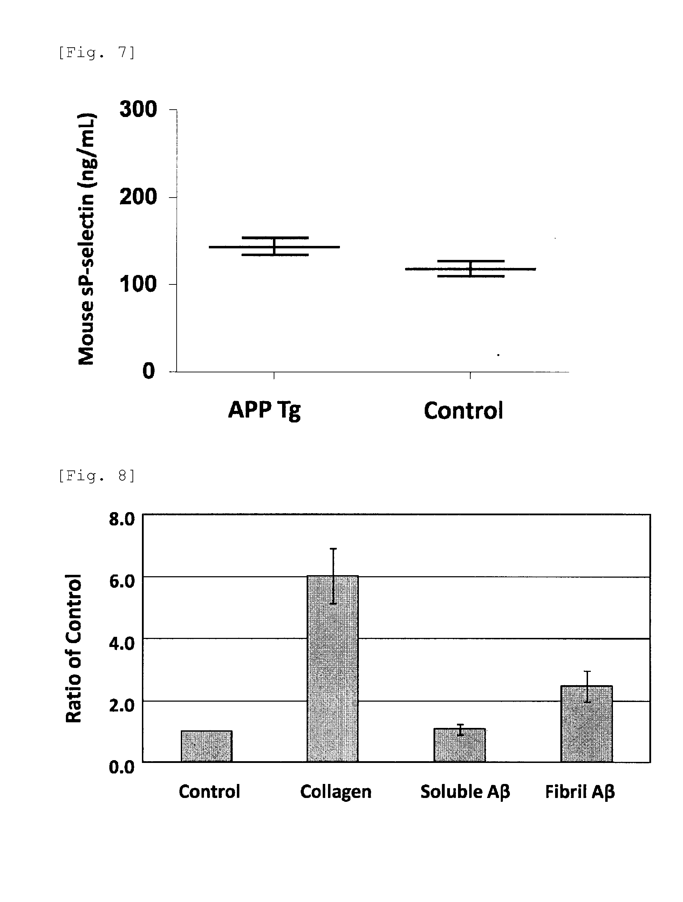

FIG. 7 is a comparative graph of the concentrations (ng/mL) of plasma sP-selectin in APP-Tg mice samples (left side of FIG. 7) using a control sample (right side of FIG. 7) of wild type mice. The long line in the figure shows the average value and the upper and lower bars indicate a standard error (SE).
FIG. 8 shows the ratio of the sGPVI production in the case where various stimulating agents such as collagen (second from the left in FIG. 8), soluble amyloid β (sAβ) (third from the left in FIG. 8), and fibril amyloid β (fAβ) were added to washed monkey platelets, when a control in the case where no stimulating agent was added was set to 1.0. The upper and lower bars indicate a standard error (SE).

### MODES FOR CARRYING OUT THE INVENTION

First, the present invention is based on the finding that the amount of sGPVI increases in a human body fluid in Alzheimer's disease. Secondly, the present invention is based on the finding that Alzheimer's disease can be diagnosed by confirming that an increase in the amount of sGPVI in the human body fluid is not caused by thrombosis or thrombotic disease.
That is, the present invention is characterized by a combination of a measurement of sGPVI in human body fluids with a measurement of a human platelet activation marker or a coagulation-fibrinolysis activation marker in human body fluids. Methods for measuring sGPVI in human body fluids have been already disclosed in a large number of documents, and for example, WO 2007/116779 and JP 2008-249552 disclose a measurement of sGPVI using an anti-GPVI antibody. As an example of a method for measuring sGPVI in human body fluids, the contents of WO 2007/116779 are herein incorporated by reference.
Also, methods to measure the activation of platelets or coagulation-fibrinolysis system in human body fluids have been disclosed in many documents, and many techniques have been already used clinically. As a method for measuring the activation of platelets or coagulation-fibrinolysis system in human body fluids, for example, a method for measuring a platelet activation marker such as sP-selectin that is a soluble form of P-selectin (P-selectin/CD62P/GMP- 140/PADGEM), a molecule of the selectin family, and a method for measuring a coagulation-fibrinolysis system activation marker, such as fibrinogen degradation product (FDP) and D-dimer in serum are exemplified.
The method of the present invention is a method for diagnosing Alzheimer's disease by combining these measurement methods. Further, the present invention provides a diagnostic agent for measuring Alzheimer's disease, comprising a combination of the measurement reagents used for these measurements.

At present, three stages of Alzheimer's disease, i.e. preclinical Alzheimer's disease, mild cognitive impairment (MCI) due to Alzheimer's disease, and Alzheimer's disease dementia are generally considered to exist (the Alzheimer's Association International Conference on Alzheimer's disease 2010 (AAICAD2010)). In the present invention, unless otherwise indicated, the Alzheimer's disease is intended to encompass all of these diseases, and includes juvenile and senile Alzheimer's diseases regardless of the ages of subject/patients. Further, it includes those complicated by non-Alzheimer's disease dementia, for example, dementia caused by Levi body disease and frontotemporal dementia such as Pick's disease, in particular, cerebrovascular dementia. The disease for the diagnosis includes preferably Alzheimer's disease dementia or MCI, particularly Alzheimer's disease dementia.

There is no particular limitation to the "human body fluid" in the present invention so long as it is a body fluid able to measure sGPVI and the activation of platelets and coagulation-fibrinolysis system, and includes a body fluid that can be collected from human blood, plasma, serum, urine, lymph, and saliva. Preferred human body fluid is blood that can be easily collected and easily measured, particularly such as plasma and serum.

As the "reagent for measuring sGPVI in a human body fluid" in accordance with the present invention, it includes a substance or a composition or a combination thereof, that can measure sGPVI in a human body fluid. Such substance may be preferably pure, but may not be necessarily pure so long as it does not affect the measurement. As a composition, a mixture of two or more substances and a conjugate, and the like are exemplified. In order to perform the measurement or in order to perform the measurement more accurately, these combinations include an aggregate of these substances or compositions when two or more substances or compositions are needed.

A preferable "reagent for measuring sGPVI in a human body fluid" of the present invention includes a substance that specifically binds to GPVI. Preferable examples of the substance that specifically binds to the GPVI include anti-GPVI antibodies. The antibodies may be polyclonal antibodies and monoclonal antibodies, but monoclonal antibodies are preferred from the viewpoint of sensitivity and specificity.
Animal species from which the anti-GPVI antibodies are derived include, for example, a mammal, particularly mouse, rat, hamster, rabbit, and the like, but are not limited thereto.
Various forms of anti-GPVI antibodies are applicable and the antibodies of the present invention include a fragment, a portion, or a derivative of antibodies, but there is no limitation to these so long as they have an ability of binding to GPVI. As an example of preferred forms, for example, the fragment includes Fab (Fragment of antigen binding), Fab', (Fab')₂, and the like, and the derivative includes a single chain antibody (scFv), a disulfide stabilized antibody (dsFv), a diabody, sc(Fv)₂ (see, for example, Orita T, Blood. 2005; 105: 562-566), a nanobody (see, for example, Cortez-Retamozo v., Cancer Research 64, 2853-2857, 2004), and a CDR-containing peptide. These may be prepared by the known methods.
The known anti-GPVI antibody includes, for example, mouse anti-human GPVI monoclonal antibody (see WO 2001/810, WO 2002/80968, WO 2005/111083), rat anti-mouse GPVI monoclonal antibody (Nieswandt et al.), rat anti-human GPVI monoclonal antibody hGP5C4 (see WO 2005/54294), human single-chain antibody (scFv) (see WO 2001/810, WO 2003/54020), and human anti-human GPVI monoclonal antibody (see WO 2005/7800), and the like.

The anti-GPVI antibody is preferably an antibody wherein the binding region and the binding site or the epitope have been identified, such as an antibody that binds to a specific domain of GPVI, for example, an antibody that binds to domain 1 or domain 2 of GPVI, or an antibody that recognizes a specific loop, for example, an antibody that recognizes at least a portion of loop 2 or loop 9 of GPVI. Specific examples include antibodies shown in Examples and antibodies described in Examples of WO 2007/116779, WO 2006/117910, and WO 2006/118350. The contents described in these patent documents are herein incorporated.
Various methods can be used to produce and identify such antibodies according to the present invention, and, while the known methods can also be applied, a preferred method uses, as its immunizing antigen or antigen for antibody identification, mutants in which a specific site on GPVI, for example, a specific domain, preferably a specific loop region, has been substituted by another amino acid sequence, specifically by the corresponding amino acid sequence from GPVI of another animal species. Specifically, the methods described in Examples of WO 2007/116779, WO 2006/117910, JP 2008-249552-A, and WO 2006/118350 can be applied. Further, the recognition regions of these antibodies can be estimated by measuring the binding between the substituted mutant and the antibody, and the binding between GPVI and the antibody in accordance with the known method. A highly specific, selective, and/or sensitive detection or determination of GPVI becomes possible by the use of one or two or more of these antibodies in which, for example, the binding site has been identified, preferably by the use of two antibodies that have different, for example, binding sites, preferably by the use of a combination of antibodies that do not compete with one another in their binding with GPVI, for example: the combination of an antibody that binds to domain 1 with an antibody that binds to domain 2, or the combination of an antibody that binds to at least a portion of loop 2 with an antibody that binds to at least a portion of loop 9. When, in particular, one or more GPVI molecular species are present as a mixture, a specific GPVI molecular species therein, for example, sGPVI, can thereby be measured, detected and determined specifically, selectively, and/or in high sensitivity.

The measurement sensitivity in the measurement of sGPVI according to the present invention is not necessarily limited, but when such a sensitivity is expressed in terms of the concentration in the sample, it may be 1 ng/mL or less or may be highly sensitive, such as 300 pg/mL or less, further 100 pg/mL or less, 30 pg/mL or less, 10 pg/mL or less, 3.0 pg/mL or less, or 1 pg/mL or less. Further, the detection limit concentration may be 1 ng/mL or less, such as 300 pg/mL or less, 100 pg/mL or less, 30 pg/mL or less, 10 pg/mL or less, 3.0 pg/mL or less, and 1.0 pg/mL or less.
Concentration in the sample from the subject may be calculated as the amount of a reference substance that is able to prepare a standard curve as shown, for example, in FIG. 1. The reference substance includes, for example, a fusion protein of sGPVI with an immunoglobulin Fc fragment (sGPVI-Fc), sGPVI to which a histidine tag (His-Tag) is bound (sGPVI-His), and the like. Also, if the operation requires dilution of the sample prior to the measurement, the value after the dilution is used.

If sGPVI in a sample of the subject, especially in plasma, is measured, it is preferable to measure GPVI specifically released from the platelets without being affected by the plasma components. Thus, a sandwich immunoassay using an antibody with a high specificity is generally used. In order to accurately measure sGPVI in the measurement method of the present invention, it is preferable to reduce the influence of the plasma components as much as possible, and dilution of the specimen is exemplified as the means for such a purpose. Further, it may be also possible to add a component such as a surfactant to the reaction mixture in order to reduce the influence of the plasma components, but such addition is not desirable when these components may have an influence on the measurement system.
The anti-GPVI antibody may be either a labeled antibody or may be a non-labeled antibody, but use of at least one labeled antibody is preferred. The labeling substance and the labeling method used for this labeling can be a known substance and a known method, and any of radioactive substances, enzymes, fluorescent substances, and chemiluminescent substances, etc., can be used and applicable. Among these, a method using an enzyme-labeled antibody is preferred because it does not require special facilities or expensive detection instrument and because it provides a convenience in handling.
Direct and indirect methods are available for the labeling, and either of these can be used.
Various antibodies can be used in use of at least one anti-GPVI antibody as the immobilized antibody in the present invention, but the immobilized antibody includes preferably an antibody that specifically binds to domain 2, more preferably to loop 9 or loop 11, particularly to loop 9. While various antibodies can be used in use as the non-immobilized antibody, the non-immobilized antibody includes preferably an antibody that specifically binds to domain 1, more preferably to loop 2 or loop 5, particularly to loop 2. In addition, among these, a combination of an antibody that specifically binds to loop 9 as the immobilized antibody and an antibody that specifically binds to loop 2 as the non-immobilized antibody is preferable.

The "reagent for measuring the activation of platelets or coagulation-fibrinolysis system in a human body fluid" of the present invention is a substance or a composition, or a combination thereof that can be used to measure the activation of platelets or coagulation-fibrinolysis system in a human body fluid. The substance is preferably pure, but it may not be necessarily pure if there is no effect on the measurement. The composition includes a mixture or a composite of two or more substances. In order to perform the measurement or in order to perform the measurement more accurately, these combinations include an aggregate of these substances or compositions when two or more substances or compositions are needed.
Preferred examples of the "reagent for measuring the activation of platelets or coagulation-fibrinolysis system in a human body fluid" of the present invention include, but not limited to, for example, platelet activation markers (e.g. P-selectin, CD40L, thromboxane B2 (TXB2), and 8-iso-prostaglandin F2α (8-iso-PGF2α), in particular, sP-selectin, β-thromboglobulin, platelet factor-4, CD63 (Lysosome-associated membrane glycoprotein 3 (LAMP-3)), sCD40L, microparticles, etc.) and coagulation-fibrinolysis system activation markers (e.g. FDP, D-dimer, thrombin-antithrombin complex (TAT), prothrombin fragment F1+2 (F1+2), etc.) in serum.
In other words, the "reagent for measuring the activation of platelets or coagulation-fibrinolysis system in a human body fluid" and the method for measuring the activation of platelets or coagulation-fibrinolysis system in a human body fluid using the reagent in the present invention are sufficient as long as it can be confirmed that the increase in the concentration of sGPVI in the human body fluid is not an increase caused by thrombotic diseases or thrombosis. Therefore, known means for measuring the activation of platelets or coagulation-fibrinolysis system in a human body fluid can be applied as they are.
As shown in FIG. 2 and FIG. 3, in any of samples of patients with Alzheimer's disease (center of FIGS. 2 and 3) and samples of patients with thrombotic disease (right side of FIGS. 2 and 3), the amount of sGPVI in the body fluid increases and it is impossible to distinguish as to whether this increase in such a state in the amount of sGPVI is due to Alzheimer's disease or due to the activation of platelets, such as thrombosis. However, by using the known diagnostic method of thrombotic diseases or thrombosis, it is possible to distinguish as to whether an increase in the amount of sGPVI in the body fluid is due to Alzheimer's disease or due to the platelet activation, for example, thrombotic disease or thrombosis.

FIG. 4 shows the results of measuring the concentration of sP-selectin in the plasma by using, as the "reagent for measuring the activation of platelets or coagulation-fibrinolysis system in a human body fluid" of the present invention, a reagent for measuring the concentration of soluble plasma P-selectin (sP-selectin) known as a kind of the platelet activation markers. As the result, the concentration of sP-selectin in the plasma has not increased in the sample of Alzheimer's patients (center of FIG. 4), but the concentration of sP-selectin in the plasma has increased in the sample of patients with thrombotic diseases (right side of FIG. 4), these facts showing that both diseases can be distinguished. Further, FIG. 5 shows the results of measuring the concentration of FDP in the serum by using, as the "reagent for measuring the activation of platelets or coagulation-fibrinolysis system in a human body fluid" of the present invention, a reagent for measuring the concentration of serum FDP known as a kind of the coagulation-fibrinolysis system activation marker. As a result, the concentration of plasma FDP in the sample of Alzheimer's patients (center of FIG. 5) has not increased, but the concentration of serum FDP in the sample of patients with thrombotic diseases (right side of FIG. 5) has increased, these facts showing that both diseases can be distinguished even by FDP measurement.
Moreover, in the studies of amyloid precursor protein transgenic mice (hereinafter described as APP-Tg mice), the similar results were obtained in comparison with the wild type (see FIGS. 6 and 7).
Since the sGPVI sandwich ELISA system also shows a cross-reactivity to monkey sGPVI in addition to human sGPVI, production of sGPVI was examined using monkey platelets. As a result, it has been suggested as one of the mechanisms producing the sGPVI that production of sGPVI may be induced due to the increase of fibril Aβ that is considered to have neurotoxic effects in patients with Alzheimer's disease (see FIG. 8).

In this way, all means for measuring the activation of platelets or coagulation-fibrinolysis system in a human body fluid can be applied to the "reagent for measuring the activation of platelets or coagulation-fibrinolysis system in a human body fluid" and the method for measuring the activation of platelets or coagulation-fibrinolysis system in a human body fluid using the reagent in the present invention so long as they may be a reagent for the measurement capable of confirming that an increase in the concentration of sGPVI in a human body fluid is not an increase due to thrombotic disease by a method other than the method for measuring an increase in the concentration of sGPVI in a human body fluid and may be a measurement method using the reagent. In other words, it may be said that a diagnostic measurement reagent capable of diagnosing thrombotic disease by a method other than the method for measuring an increase in the concentration of sGPVI in a human body fluid and a measurement method using the reagent are all included in the "reagent for measuring the activation of platelets or coagulation- fibrinolysis system in a human body fluid" and the method for measuring the activation of platelets or coagulation- fibrinolysis system in a human body fluid using the reagent in the present invention.

Various known measurement principles can be all applied to the reagent for measuring sGPVI in a human body fluid and the method using the reagent according to the present invention, as well as to the reagent for measuring the activation of platelets or coagulation-fibrinolysis system in a human body fluid and the measurement method using the reagent according to the present invention, but an immunoassay method is generally preferred. Examples of the immunoassay method include, for example, enzyme-antibody methods, ELISA methods, sandwich immunoassay methods, agglutination methods, solid-phase direct methods, solid-phase binding methods, solution reaction methods, competitive assays, non-competitive assays, immunochromatographic methods, and flow-through methods; these methods can be applied alone or in combination thereof (see, for example, "Ultrasensitive Enzyme Immunoassay Methods", Eiji ISHIKAWA, Japan Scientific Societies Press (1993); "New Uses of Immunoassay Methods and Their Applications to the Development of Diagnostic Reagents and Therapeutic Drugs", Association for Research and Development in Immunoassay Methods, Keiei Kyoiku Publishing; and Enzymatic Immunoassay Methods (Third Edition), edited by Eiji ISHIKAWA et al., Igaku Shoin (1987)). The following can also be used: Measurement by the MEDIA method (JP 5-264552-A), which employs an electrochemical measurement of signals from the label; Immunoassays using a microchip ("Bioscience and Industry", Volume 61 (2003), pp. 449-454); Time-resolved immunofluorometric assays ("Analytical Biochemistry" (USA), Volume 137 (1984), pp. 335-343); and Homogeneous immunoassay methods. Among these methods, sandwich immunoassay methods, particularly sandwich ELISA methods that use an insoluble carrier, e.g., a microwell plate, and the like are convenient and are also preferred from the standpoint of sensitivity.

The known technology can be employed for the sandwich immunoassay method. The principles and applications of this measurement method and improvements thereto are described in, for example, "Ultrasensitive Enzyme Immunoassay Methods", Eij i ISHIKAWA, Japan Scientific Societies Press (1993); "New Uses of Immunoassay Methods and Their Applications to the Development of Diagnostic Reagents and Therapeutic Drugs", Association for Research and Development in Immunoassay Methods, Keiei Kyoiku Publishing; and Enzymatic Immunoassay Methods (Third Edition), edited by Eiji ISHIKAWA et al., Igaku Shoin (1987), each of which is incorporated herein by reference. The sandwich immunoassay method is generally a method in which measurement is carried out by the formation of an antibody-antigen-antibody complex using at least two antibody species that differ in their recognition sites for the protein to be measured. The sandwich immunoassay method usually employs an insoluble carrier, and in such a case the first antibody, which is bonded to the insoluble carrier, is sometimes referred to as an immobilized antibody or as an antibody for immobilization or as a capture antibody, while the second antibody is sometimes referred to as a non-immobilized antibody, a liquid-phase antibody, a detection antibody, or a labeled antibody when directly or indirectly labeled. An insoluble carrier having the first antibody bonded thereto is first prepared to provide a solid-phase or a reaction site. The specimen is added to this solid-phase insoluble carrier to carry out the reaction. After reaction for a prescribed period of time, washing is carried out to remove substances that have not specifically bound to the solid phase. The labeled second antibody is then added. After reaction for a prescribed period of time, washing is done to remove the labeled antibody that has not participated in complex formation, and, based on the label, the amount of complex specifically bound to the solid phase is specifically determined qualitatively or quantitatively. This sandwich method can be carried out by a procedure in which two steps are implemented as described above (two-step method) or by a single step procedure in which the antigen and the labeled antibody are added at the same time (one-step method).

The sandwich immunoassay method can also be carried out in solution without using an insoluble carrier. For example, there is a procedure in which an antigen is reacted in the liquid phase with a labeled antibody and a second binding substance tagged with a second label so that the interaction between the label and the second label is measured.
Moreover, in the sandwich immunoassay method, measurement as an alternative method can also be carried out by utilizing a second specific binding. This is a method in which the measurement is carried out by forming a complex comprising antibody-antigen-antibody-second specific binding substance or by forming a complex comprising antibody-antigen-antibody-second specific binding substance-specific binding partner for the second specific binding substance (hereinafter sometimes described as a second specific binding partner). The second specific binding substance-second specific binding partner combination can be an antigen and its antibody; a ligand and its receptor; a sugar chain-containing substance and lectin; biotin and avidin or streptavidin; and the like. Additional examples are as follows: procedures that carry out a measurement using an antibody against an antibody, i.e., an anti-immunoglobulin antibody, to form an antibody-antigen-antibody-anti-immunoglobulin antibody complex, and procedures that carry out a measurement using an anti-immunoglobulin antibody and a second specific binding to form an anti-immunoglobulin antibody-antibody-antigen-antibody-second specific binding substance-second specific binding partner and the like.
In addition, in the immunoassay method, a competitive method can also be used as an alternative measurement method. This is a method in which measurement is carried out based on the competition, during the formation of the antigen-antibody complex, between an antigen in the sample and a labeled antigen or a labeled antigen-like substance.

As a preferred example of the present invention that employs two kinds of antibodies having different binding sites, a system is exemplified, in which using biotin and avidin or streptavidin as a second specific binding substance-second specific binding partner, an antibody, especially a non-immobilized antibody is biotinylated and detected with a labeled streptavidin, particularly with a poly-HRP-labeled streptavidin.
The insoluble carrier used in the sandwich immunoassay system may be beads, latex particles, magnetic particles, plate, tube, membrane, and the like. The material of the beads, plate, or tube can be, for example, polystyrene, nylon, glass, silicone rubber, stainless steel, plastic, and the like. The membrane includes, for example, cellulose, cellulose derivative, nitrocellulose, porous synthetic polymer, glass fiber, fabric, nonwoven fabric, filter paper, and the like. With regard to shape, a spherical shape can be used for the beads, latex particles, magnetic particles, and the like, and this is advantageous in terms of preserving space during storage. A well shape can be used for the plate and tube, and this is advantageous in terms of being applicable to commercial automatic assay instrumentation, plate readers, and the like. A membrane can also be used in immunochromatographic and flow-through methods. Bonding to the insoluble carrier of the antibody, second binding substance, second specific binding substance or its partner, or anti-immunoglobulin antibody can be effected by, for example, thermal adsorption, chemical bonding, and the like.
In order to reduce reactions such as nonspecific adsorption and so forth and raise the specificity or sensitivity of the measurement system, a blocking treatment with a substance that does not influence the measurement system is preferably carried out on its non-adsorbing surfaces of the insoluble carrier, where the aforementioned substances are not bound. The following are examples of the substance that does not influence the measurement system: proteins such as bovine serum albumin (BSA) and casein, and surfactants such as Tween 20 and NP-40.

The label used in the sandwich immunoassay system kit can be exemplified by enzymes such as peroxidase, particularly horseradish peroxidase (HRP), alkaline phosphatase, β-D-galactosidase, oxidase, uricase, etc.; chemiluminescent substances such as acridinium or derivatives thereof, aequorin or variants thereof, etc.; fluorescent substances e.g. FITC, lanthanoid, such as europium (Eu), samarium (Sm), etc.; as well as dyes, colloidal gold, colored latexes, isotopes, etc.
For example, when a peroxidase is used as the enzyme, there are exemplified by 3,3',5,5'-tetrabenzidine and 1,2-phenylenediamine as a chromogenic substrate; when an alkaline phosphatase is used as the enzyme, there is exemplified by 4-nitrophenyl phosphate as a chromogenic substrate; and when β-D-galactosidase is used as the enzyme, there is exemplified by 2-nitrophenyl-β-D-galactoside as a chromogenic substrate.
Labeling with the enzyme of the antibody, second binding substance, second specific binding substance or partner thereof, or anti-immunoglobulin antibody can be carried out by the two-step glutaraldehyde method, periodic acid method, maleimide method, pyridyl disulfide method, and the like. For non-enzyme labels, this can be done using the known art, e.g., thermal adsorption, chemical bonding, and the like.
The use of an enzyme label with a chromogenic substrate as exemplified above makes it possible to carry out the measurement using the usual absorbance measurement systems and also provides a relatively high sensitivity and for these reasons is preferred. The measurement can be carried out by a measuring instrument conforming to the particular label when a chemiluminescent substance, fluorescent substance, colored label, or isotope is used as the label. In addition, when a fluorescent substance such as Eu, for example, a cryptate (Eu³⁺ cryptate), is used, the fluorescent resonance energy transfer can be measured using an allophycocyanine derivative, for example, XL665, as a second label. Dyes, colloidal gold, and colored latices can also be visually evaluated and for this reason are preferred for the label used in easy-to-use measurement kits, for example, kits that employ an immunochromatographic method or a flow-through method. The particles used as the insoluble carrier in an agglutination method can be those particles that are ordinarily used, for example, latices, erythrocytes (for example, sheep erythrocytes), gelatin, microbeads, carbon particles, and the like.

The measurement reagent or kit of the present invention may contain optional constituent components or constituent elements in addition to at least an active substance for the measurement. The optional constituent components in the measurement reagent or kit can be exemplified by additives such as stabilizers, vehicles, preservatives, etc., while the optional constituent elements in the kit can be exemplified by buffer solutions or diluents for the standard substance, specimen, labeled antibody, and the like; chromogenic substrate adapted to the enzyme when an enzyme is used in the labeled antibody; blocking agent; reaction stopper; rinsing agent; and the like. While the diluent is not particularly limited, a diluent containing a substance present in the specimen is preferred. When the specimen is serum and the blood draw to acquire the serum has been carried out in the presence of EDTA or citric acid, the same amount of EDTA or citric acid is preferably also present in the diluent. For example, 0.2 to 1 mg/mL EDTA is preferably present in the diluent. The standard substance is, for example, a standard reference prepared from a biological sample or a recombinant product prepared by genetic engineering. These can be prepared by the known methods.

The present invention provides a diagnostic agent capable of noninvasively, easily, and specifically determining or diagnosing Alzheimer disease, without the need for specialized instrument, through measurement of sGPVI or the activation of platelets or coagulation-fibrinolysis system in a human body fluid collected as a specimen from humans, by using a measurement reagent able to measure sGPVI in a human body fluid collected as a specimen from humans and a measurement reagent able to measure the activation of platelets or coagulation-fibrinolysis system in the human body fluid, or provides a kit for use in diagnosing Alzheimer disease.
The diagnostic method of the present invention comprises collecting a body fluid from the subject, optionally diluting the collected sample of the subject to a concentration that can be measured, measuring the concentration of sGPVI in the sample by using the measurement reagent of the present invention, measuring simultaneously or sequentially the activation of platelets or coagulation-fibrinolysis system in the sample by using the measurement reagent of the present invention for measuring the activation of platelets or coagulation-fibrinolysis system, and comparing the measured values with those of the control group of healthy individuals, preferably with age-matched values or values of healthy individuals of the same age.
Comparison with the value measured for healthy individuals is performed as follows. In an example of this step, the results of the measurement in a plurality of healthy individuals are obtained in advance; a standardized healthy value or healthy range, obtained by taking the average of these measurement results or a range of these measurement results, is made the standard healthy value as a control; and this is compared with the measured value. For example, in this comparison, using the standard deviation (SD) or standard error (SE), the standard healthy value for healthy individuals may be acquired by designating the average value for healthy individuals + 2 SD (or SE) or 3 SD (or SE) as the cutoff value. In addition, a step may be implemented in which a reference value is established in advance for the patients of a disease and compared with the measured value. In addition, a statistical significant difference can also be used as an index.
In a certain aspect, it is useful to observe the changes over time of the same person, not to make a comparison with the control. For example, by performing an inspection at intervals of about 10 years, 5 years, 3 years, 1 year, or 6 months, an early or accurate diagnosis, or a decision of the stage or progress of disease becomes possible.

Hereinafter, the present invention will be described in more detail by way of Examples, but it shall not be limited in any way by these Examples.

### Example 1

### ● Preparation of F1232-10-2 Fab'-HRP

F1232-10-2 F(ab')₂ was prepared by treating anti-GPVI antibody F1232-10-2, described in Examples of WO 2007/116779 and WO 2006/118350, with lysylendopeptidase.
In other words, 1 AU of lysylendopeptidase (manufactured by Wako) was added to 50 mg of F1232-10-2 after buffer exchange into Phosphate-Buffered Saline D-PBS (SIGMA). After 4 hours of the reaction at 37°C, a serine protease inhibitor, tosyllysine chloromethylketone (TLCK, manufactured by SIGMA) was added to the reaction mixture to a final concentration of 30 mM, thereby to stop the reaction. Then, F1232-10-2 F(ab')₂ was purified. That is, in order to remove the cleaved Fc portion and the uncleaved F1232-10-2, an enzyme-digested antibody was applied to a Protein A column (manufactured by Millipore). The unbound fraction containing F(ab')₂ was dialyzed and subjected to buffer exchange into 4 mM Tris-HCl (pH 8.5). Then, the solution was subjected to an anion-exchange chromatography using Mono Q column (manufactured by GE Healthcare). The obtained F1232-10-2 F(ab')₂ was buffer exchanged into D-PBS and the protein concentration was measured by Protein Assay Dye Reagent(manufactured by Bio-Rad) using bovine serum albumin as a standard.
Subsequently, F1232-10-2 F(ab')₂ was labeled with HRP using a peroxidase labeling kit SH (manufactured by Dojindo Laboratories). That is, F1232-10-2 F(ab')₂ was partially reduced so that the cysteine residue present in the hinge region was labeled with peroxidase.

### Example 2

### ● sGPVI sandwich ELISA system

Anti-GPVI antibody F1232-7-1 described in Examples of WO 2007/116779 and WO 2006/118350 was prepared into 10 µg/ml using D-PBS, applied to an immunoplate (C8 Maxisorb, manufactured by Nunc) in 50 µl/well, and incubated overnight at 4°C. The immobilized plate was washed five times with ice-cold ion-exchanged water, and 5% Stabiolguard, 0.1% Tween 20 and 3.2% sucrose/D-PBS were added in 200 µl/well to block the unreacted portion. Then, the plate was refrigerated and stored under vacuum drying.
Human serum and human plasma were prepared by a 100-fold or more dilution with 0.1% BSA, 0.05% Tween 20, and 0.3 M NaCl/D-PBS and added to the F1232-7-1-immobilized plate to incubate at 28°C for 2 hours. In addition, a recombinant human sGPVI-His that was expressed and purified by Free Style 293 Expression System (manufactured by Invitrogen) was used as the reference material. After the reaction incubation, the plate was washed with physiological saline containing 0.05% Tween 20. F1232-10-2 Fab'-HRP prepared in Example 1 as a secondary labeled antibody was diluted 100-fold with 2% rat serum and 0.05% Tween 20/D-PBS and added to the well to incubate at 28°C for 1 hour. After washing the plate, tetramethylbenzidine (hereinafter described as TMB) that was returned to room temperature was added, and incubated for 10 minutes. After stopping the reaction by the addition of 0.5 M sulfuric acid, the absorbance at 450 nm was measured using a microplate reader SpectraMax (manufactured by Molecular Devices), and the absorbance at 650 nm was subtracted as a reference. The resulting absorbance was analyzed using a microplate data analysis software (SoftMax Pro 5.2, manufactured by Molecular Devices). A standard curve was shown in FIG. 1. In addition, the data was expressed in terms of mean ± standard error (SE) in principle.

### Example 3

### ● Measurement of serum and plasma level of sGPVI in patients with each disease

The sGPVI contained in serum and plasma of patients with each disease was measured using a sandwich ELISA system prepared in Example 2. Samples from patients with thrombotic disease, samples from patients with Alzheimer's disease, and control samples from healthy individuals were assayed. In addition, healthy individual sample was matched for age to the elderly Alzheimer's patient.
The measurement results using a plasma sample were shown in FIG. 2, and the measurement results using a serum sample were shown in FIG. 3. The concentrations of sGPVI in serum and plasma of patients with thrombotic disease and patients with Alzheimer's disease were higher than those of the control group of healthy individuals.

### Example 4

### ● Measurement of plasma level of sP-selectin in patients with each disease

The plasma concentration of sP-selectin, platelet activation marker, in each disease used in Example 3 was measured with a GMP-140 (P-selectin) EIA kit (TAKARA). The measurement results were shown in FIG. 4. The concentration of sP-selectin in patients with thrombotic disease was higher than in healthy individuals as a control group, but no difference was observed between patients with Alzheimer's disease and healthy individuals as a control group.

### Example 5

### ● Measurement of serum level of fibrinogen degradation products (FDP) in patients with each disease

The Serum level of FDP, coagulation-fibrinolysis system activation marker, in each disease used in Example 3 was measured with a FDP measurement kit LATECLE FDP (manufactured by Kainos Laboratories, Inc.). The results were shown in FIG. 5. The concentration of fibrinogen degradation products (FDP) in patients with thrombotic disease was higher than in healthy individuals as a control group, but no difference was observed between patients with Alzheimer's disease and healthy individuals as a control group.

### Example 6

### ● Comparison of sGPVI concentration in patients with each disease with platelet activation marker and coagulation-fibrolysis system activation marker

The results of Examples 3 to 5 were analyzed using a statistical software SAS/STAT 9.1.
First analysis was perfomed for Plasma samples. That is, the concentrations of sGPVI in plasma samples of patients with each disease, measured in Example 3, were analyzed using Dunnett-type Steel's test of nonparametric multiple comparison procedures. The sGPVI level in an Alzheimer's patient group (age 88.9 ± 4.5) and a thrombotic disease patient group (age 67.1 ± 10. 9) were compared to a healthy individual control group (age 84.5 ± 7.1). As a result, the sGPVI concentrations of both the Alzheimer's patient group (p = 0.00044) and the thrombotic disease patient group (p = 0.0024) were significantly elevated. In addition, statistical analysis was performed for the plasma sP-selectin levels in a similar manner, measured in Example 4, in patients with each disease. As a result, the sP-selectin concentration of the thrombotic disease patient group was significantly elevated (p = 0.0033) in comparison with the healthy individual control group, whereas no difference was observed between the Alzheimer's patient group and the healthy individual control group (p = 0.96).
Then serum samples were analyzed. The concentrations of sGPVI in serum samples of patients with each disease, measured in Example 3, were analyzed using Dunnett-type Steel's test of nonparametric multiple comparison procedures. The sGPVI concentrations between in the Alzheimer's patient group (age 85.1 ± 11.4) and the thrombotic disease patient group (age unknown) were compared to the healthy individual control group as the control group (age 84.6 ± 9.1). As a result, the sGPVI concentrations of both the Alzheimer's patient group (p = 0.027) and the thrombotic disease patient group (p = 0.045) were significantly elevated. In addition, when statistical analysis was performed for the serum FDP concentrations in a similar manner, measured in Example 5, in patients with each disease, the FDP concentration of the thrombotic disease patient group was significantly elevated (p = 0.018) in comparison with the healthy individual control group, whereas no difference was observed between the Alzheimer's patient group and the healthy individual control group (p = 0.41). These results indicate that Alzheimer's disease can be specifically detected by combination of sGPVI and a platelet activation marker such as sP-selectin, or sGPVI and a congealing fibrinogenolysis system activation marker such as FDP.

### Example 7

### ● Establishment of mouse sGPVI sandwich ELISA system (1) Generation of rabbit anti-mouse GPVI antibody

Recombinant mouse sGPVI-His (85 µg) was dissolved in 500 µl of saline, mixed with an equal volume (500 µl) of Freund's complete adjuvant (DIFCO) and administered subcutaneously to the back of female 10-week-old New Zealand white rabbits (Kitayama Labes). Two weeks later, 85 µg of recombinant mouse sGPVI-His was dissolved in 500 µl of saline, mixed with an equal volume (500 µl) of Freund's incomplete adjuvant (DIFCO) and administered subcutaneously to the back of the rabbits.
After one week from the second administration, blood was collected from the rabbit ear artery, and antiserum was separated, followed by purification of antibody. Ammonium sulfate was added to the antiserum so that the final saturated concentration became 50%, and the mixture was stirred at room temperature for 1 hour. The ammonium sulfate precipitation was separated by centrifugation. The precipitate fraction was dissolved with a phosphate buffer (SIGMA) (hereinafter described as D-PBS) and further buffer exchanged into D-PBS by a dialysis method. After filtration of the dialyzed sample, the filtrated sample was applied to Protein A column (manufactured by Millipore), and the bound fraction was eluted with 0.1 M glycine-HCl buffer (pH 3.0). The pH of the immunoglobulin fraction eluted was neutralized by the addition of a 1/10-fold amount of 1 M Tris buffer (pH 8.5) and buffer exchange into D-PBS was performed by a dialysis method. The concentration of protein of the rabbit anti-mouse GPVI antibody was measured with Protein Assay Dye Reagent (manufactured by Bio-Rad) using bovine serum IgG as a standard.

### (2) Preparation of rabbit anti-mouse GPVI antibody Fab'-HRP

The rabbit anti-mouse GPVI antibody prepared in the above (1) was buffer exchanged into an acetic acid buffer solution (pH 4.0), and pepsin (Roche) was added in the weight ratio of 30 : 1 (antibody : pepsin). After 3 hours of the reaction at 37°C, the reaction mixture was adjusted to a neutral pH by the addition of 2 M Tris buffer (pH 8.5), so that the reaction was stopped.
Then, the antibody that had been digested with pepsin was buffer exchanged into 4 mM Tris buffer (pH 8.5) and subjected to a chromatography on an anionic column (Mono Q Column) (manufactured by GE Healthcare). The purified F(ab')₂ fraction was buffer exchanged into D-PBS, and the protein concentration was measured in the same manner as described above (1).
Next, the rabbit anti-mouse GPVI antibody F(ab')₂ was partially reduced, and the cysteine residue present in the hinge region was labeled with peroxidase using a Peroxidase Labeling Kit SH (manufactured by Dojindo Laboratories), thereby to prepare a labeled antibody (indicated as rabbit anti-mouse GPVI antibody Fab'-HRP).

### (3) Establishment of mouse sGPVI sandwich ELISA system

The rabbit anti-mouse GPVI antibody made in the above (1) was prepared into a 10 µg/ml solution using D-PBS. The antibody solution prepared by dilution was applied into an immunoplate (C8 Maxisorb, manufactured by Nunc) in an amount of 50 µl/well. The plate was incubated overnight at 4°C, the immobilized plate was washed five times with ice-cold ion-exchanged water, and 2% StabilGuard, 0.1% Tween 20 and 3.2% sucrose/D-PBS were added in 200 µl/well to block the unreacted portion. Then, the plate was stored under vacuum drying.
Mouse plasma was prepared by a 10-fold or more dilution with 0.1% BSA and 0.05% Tween 20/D-PBS and added to the rabbit anti-mouse GPVI antibody immobilized plate, and the plate was incubated at room temperature for 2 hours. In addition, a recombinant mouse sGPVI-His was used as a reference material. After the incubation, the plate was washed with a saline containing 0.05% Tween 20. The rabbit anti-mouse GPVI antibody Fab'-HRP made in the above (2) as a secondary labeled antibody was diluted 100-fold with 2% rabbit serum and 0.05% Tween 20/D-PBS, and added to the plate and the plate was incubated at room temperature for 2 hours. After washing the plate, TMB that was returned to room temperature was added and incubated for 10 minutes. After stopping the reaction by the addition of 0.5 M sulfuric acid, the absorbance at 450 nm was measured using a microplate reader SpectraMax (manufactured by Molecular Devices), and the absorbance at 650 nm was subtracted as a reference. The absorbance obtained was analyzed using a microplate data analysis software SoftMax Pro 5.2 (manufactured by Molecular Devices). A standard curve was prepared in a similar manner to Example 2 described above.

### Example 8

### ● Study on amyloid precursor protein transgenic mice (hereinafter described as APP-Tg mice)

### (1) Measurement of plasma sGPVI concentration

Using a sandwich ELISA system prepared in Example 7, the plasma sGPVI level in 18- to 24-month-old APP-Tg mice (B6; SJL-Tg (APPSWE) 2576Kha, Taconic Farms Inc.) and control mice was measured. In addition, wild-type mice with the same genetic background were used as the control. The measurement results were shown in FIG. 6.

### (2) Measurement of concentration of mouse plasma sP-selectin

Using the plasma of each mouse used in Example 8, the concentration of sP-selectin, a platelet activation marker was measured. An sP-selectin ELISA Kit (manufactured by R&D) was used for the measurement. The measurement results were shown in FIG. 7.

### (3) Comparison of the level of sGPVI and the level of platelet activation marker in each mouse

By using a statistical software SAS/STAT 9.1, the plasma sGPVI level measured in the above (1) in each mouse were analyzed by Wilcoxon rank sum test. As a result, the sGPVI concentration was significantly increased in the APP-Tg mice group (P = 0.0011). In addition, when the plasma level of sP-selectin measured in the above (2) in each mouse were analyzed by Wilcoxon rank sum test, no significant difference was observed between the two groups (P = 0.0941).
These results indicate that sGPVI in Alzheimer's disease mouse models is produced without any platelet activation, as well as in human Alzheimer's patients.

### Example 9

### ● Study on production mechanism of sGPVI

The sandwich ELISA system shown in Example 2 also has a cross-reactivity to monkey sGPVI in addition to human sGPVI. Consequently, production of sGPVI was examined using monkey platelets.

### (1) Preparation of washed monkey platelets

Whole blood was collected from the monkey' s leg vein using sodium citrate as an anticoagulant. Platelet-rich plasma (hereinafter described as PRP) was prepared by centrifuging the obtained whole blood at 900 rpm × 10 minutes, and only PRP fraction was separated gently by PIPETMAN to transfer it to a new tube. The PRP was centrifuged at 2000 rpm × 10 minutes at room temperature to precipitate the platelets. The precipitated platelets were suspended in 0.02% BSA/10 mM HEPES buffer solution (pH 6.4) containing ACD (acid-citrate-dextrose)-A solution that had been prepared according to a conventional method, and centrifuged again at 2000 rpm × 10 minutes to precipitate the platelets. This operation for the washing purpose was performed twice. After completion of the second wash, platelets were precipitated by centrifugation, and stirred in 0.02% BSA/HEPES buffer (pH 7.4) . The number of platelets was counted by Sysmex F-820 (Toa Medical Electronics Co., Ltd.), and the platelet count was adjusted to 40.0 × 10⁷/ml. These platelets were served as washed platelets.

### (2) Preparation of amyloid β (hereinafter described as Aβ)

Soluble amyloid β (hereinafter described as sAβ) and fibril amyloid β (hereinafter described as fAβ) were prepared by the following procedure according to Cindy M Sondag' s method (Journal of Neuroinflammation 2009 Jan 5; 6:1-13).
Aβ1-42 (AnaSpec Inc) was adjusted to become 1 mg/mL by the addition of hexafluoroisopropanol (SIGMA) and incubated at room temperature for 1 hour to completely dissolve. After complete evaporation of the hexafluoroisopropanol by gentle warming, the residue was dissolved in dimethyl sulfoxide (DMSO) so that Aβ1-42 became 10 mg/mL, and further diluted to 400 µg/mL with D-PBS. Aβ just after diluted in D-PBS was used as sAβ, and Aβ that was incubated at room temperature for one week after the dilution was used as fAβ.

### (3) Study on sGPVI production mechanism using monkey washed platelets

The production of sGPVI was examined by adding various stimulating agents such as collagen, sAβ, or fAβ, to monkey washed platelets. That is, 4 µL of 100 mM calcium chloride solution was added to 180 µL of the washed platelet suspension prepared in the above (1). Furthermore, 20 µL of each stimulus agents was added, and incubated at 37°C for 1 hour. After the incubation, the reaction mixture was centrifuged at 15000 rpm × 2 minutes, the supernatant was transferred to a new tube, and Protease Inhibitor Cocktail (SIGMA) and EDTA were added to a final concentration of 5% and 5 mM, respectively, thereby to stop the reaction. The concentration of sGPVI in the reaction mixture was measured by the ELISA method as described in Example 2. The level of sGPVI produced by various stimulus was showen in Fig. 8, and the condition of no stimulus was showed as control.
As shown in FIG. 8, when compared with the control group without addition of any stimulating agents, production of sGPVI was observed in the case of addition of collagen, ligand of GPVI, . On the other hand, even in the case where sAβ was added, production of sGPVI was not observed, but in the case of addition of fibril Aβ, considered to have a neurotoxic effect in patients with Alzheimer's disease, production of sGPVI was observed.
This result showed that fibril Aβ, neurotoxic effect in patients with Alzheimer's disease, would be a possibility of inducing the production of sGPVI.

### INDUSTRIAL APPLICABILITY

The present invention provides a diagnostic agent that can diagnose Alzheimer's disease noninvasively and easily without the need for specialized instruments, or a kit for use in the diagnosis, and they are industrially useful as diagnostic agents or diagnostic kits.

## Claims

1. A reagent for measuring soluble GPVI (sGPVI) in a human body fluid; and a diagnostic agent for Alzheimer's disease or its risk, comprising a reagent for measuring the activation of platelets or coagulation-fibrinolysis system in the human body fluid, or a screening agent therefor.

2. The diagnostic agent or screening agent according to claim 1, wherein the reagent for measuring sGPVI is a reagent comprising at least a kind of anti-GPVI antibodies.

3. The diagnostic agent or screening agent according to claim 1 or 2, wherein the reagent for measuring the activation of platelets or coagulation-fibrinolysis system is a reagent for measuring a platelet activation marker and/or a coagulation-fibrinolysis system activation marker.

4. The diagnostic agent or screening agent according to any of claims 1 to 3, wherein the platelet activation marker is soluble P-selectin.

5. The diagnostic agent or screening agent according to any of claims 1 to 4, wherein the coagulation-fibrinolysis system activation marker is a fibrinogen degradation product (FDP) or D-dimer in serum.

6. A method for screening, determining, or diagnosing Alzheimer's disease or its risk, the method comprising a process for measuring the concentration of sGPVI in a collected human body fluid and a process for measuring the activation of platelets or coagulation-fibrinolysis system in the human body fluid.

7. The method according to claim 6, wherein the process for measuring the concentration of sGPVI comprises at least one of anti-GPVI antibodies.

8. The method according to claim 6 or 7, wherein the process for measuring the activation of platelets or coagulation-fibrinolysis system is a method for measuring a platelet activation marker and/or a coagulation-fibrinolysis system activation marker.

9. The method according to any of claims 6 to 8, wherein the platelet activation marker is sP-selectin.

10. The method according to any of claims 6 to 9, wherein the coagulation-fibrinolysis system activation marker is a fibrinogen degradation product (FDP) or D-dimer in serum.
